# EUROPEAN PATENT APPLICATION

(11) **EP 2 683 198 A1**
(43) Date of publication of application: **08.01.2014**
(21) Application number: 13175440.0
(22) Date of filing: 05.07.2013
(51) Int. Cl.: H04W 40/12, H04W 40/22, H04L 12/721, H04L 12/707, H04L 12/735

(54) **A wireless network System, a Method and a Computer-readable Medium for setting up the System.**

(30) Priority: 05.07.2012 US 201261668066 P; 28.08.2012 SE 1250956; 14.09.2012 SE 1251027
(71) Applicant: Captera AB, 24643 Löddeköpinge (SE); Isonova VOF, 6074CS Melick (NL)
(72) Inventor: Verstraelen, Hans, 6077CV St.Odilienberg (NL)
(74) Representative: KIPA AB

(57) **Abstract**

The disclosure is related to a wireless network system. In particular, the disclosure relates to a method of setting up such a wirleless netwok system. The disclosure provides for an improved and more reliable monitoring system. In one embodiment, a monitoring system is provided, which comprises a first gateway (202); optionally a second gateway (204); a plurality of transceivers; and wherein a preferred route of transmitting between the first gateway (202) and a transceiver is selected for each transceiver, the preferred route being either directly between the first gateway (202) and the transceiver or between the first gateway (202) and the transceiver via at least one preferred route intermediate transceiver depending on a value of signal quality or signal strength, such as a Received Signal Strength Indicator (RSSI), of the transceiver measured at the first gateway (202) or a value of signal quality or signal strength, such as an RSSI, of the gateway measured at the transceiver

## Description

### BACKGROUND OF THE INVENTION

### Field of the Invention

This invention pertains in general to the field of a wireless network system. More particularly, the invention relates to a method of setting up a a wireless network system. Especially a wireless network system to be used, for example, in a patient monitoring system or in a social alarm monitoring system or in a mentally disabled people monitoring system, such as dementry, wandering people etc.

### Description of the Prior Art

There are some wireless network systems, such as patient monitoring and patient alarm systems known from prior art. As an example, the prior art document US2011/0211563 A1 discloses a location tracking system, which includes handling of patient alarms. From paragraph [0022] of the document, it is clear that signals between a server and a plurality of user equipment can be routed through a plurality of base stations. The network can be based on Bluetooth. However, from this document it is not clear how the routing is performed. Furthermore, the document does not disclose anything about any redundancy in the system in order to make it more reliable. Moreover, a system, such as the system disclosed in the prior art document, may have problems with dead-spots, i.e. locations where no signal can be obtained.

Hence, an improved wireless networking system application, would be advantageous.

In addition, a system with optimized routing of signals may be advantageous.

Furthermore, a more reliable patient monitoring system, or social alarm monitoring system, or mentally disabled people monitoring system may be advantageous. Moreover, a wireless networking system without dead-spots, i.e. locations where no signal can be obtained, may be advantageous.

### SUMMARY OF THE INVENTION

Accordingly, embodiments of the present invention preferably seek to mitigate, alleviate or eliminate one or more deficiencies, disadvantages or issues in the art, such as the above-identified, singly or in any combination by providing a wireless networking system system, a method and a computer-readable medium for setting up the system, according to the appended patent claims. Particularly a wireless networking system to be used, for example, in a patient monitoring system or in a social alarm monitoring system or in a mentally disabled people monitoring system, such as dementry, wandering people etc.

According to aspects of the invention, a wireless networking system, a method and a computer-readable medium for setting up the system are disclosed. The system may be used to obtain an improved and more reliable, for example, patient monitoring system or in a social alarm monitoring system or in a mentally disabled people monitoring system, such as dementry, wandering people etc.

According to one aspect of the invention, a wireless networking system is provided. The system comprises a first gateway. Optionally, the system further comprises a second gateway. Moreover, the system also comprises a plurality of transceivers. In the system, a preferred route of transmitting between the first gateway and a transceiver is selected for each transceiver. The preferred route can be either directly between the first gateway and the transceiver. As an alternative, the route can be between the first gateway and the transceiver via at least one preferred route intermediate transceiver. A decision regarding whether the preferred route is directly between the first gateway and the transceiver or via at least one preferred route intermediate transceiver is based on a value of signal quality or signal strength of the transceiver measured at the first gateway or a value of signal quality or signal strength of the gateway measured at the transceiver. The measured value of the signal strength may be a Received Signal Strength Indicator (RSSI).

According to another aspect of the invention, a method of setting up a wireless networking system is provided. The method comprises an optional step of mapping rooms, of a group of rooms, with transceivers of a first group of transceivers. The method further comprises selecting a gateway as a current node. Furthermore, the method comprises broadcasting an installer command from the current node. Moreover, the method comprises waiting, with the current node, for a present group of transceivers to report as a response to the installer command. The transceivers of the present group are not linked to the gateway. The present group of transceivers belongs to the first group of transceivers. In addition, the method comprises measuring signal quality or signal strength of each transceiver of the present group of transceivers. The method further comprises comparing measured values of signal quality or signal strength, such as Received Signal Strength Indicators (RSSI), of transceivers in the present group of transceivers. Furthermore, for each transceiver of the present group of transceivers, a link between the current node and the transceiver of the present group of transceivers is established, if a criterion is met. A criterion that can be used is if the RSSI of the transceiver is higher than a threshold. The method also comprises, selecting, from the present group of transceivers, a transceiver with a best signal quality or a largest signal strength, such as a largest RSSI, as a current node. Finally some of the above given steps are repeated until a preferred route of transmitting from the gateway to each transceiver has been established.

According to a further aspect of the invention, a computer-readable medium having embodied thereon a computer program for processing by a computer is provided. The computer program comprises an optional first code segment for mapping a group of rooms with a first group of transceivers. The computer program further comprises a second code segment for selecting a gateway as a current node. Furthermore, the computer program comprises a third code segment for broadcasting an installer command from the current node. Moreover, the computer program comprises a fourth code segment for, with the current node, waiting for a present group of transceivers, transceivers of the present group not being linked to the gateway, the present group of transceivers belonging to the first group of transceivers, to report as a response to the installer command. In addition, the computer program comprises a fifth code segment for receiving from the server 200 a measured signal quality or signal strength of each transceiver of the present group of transceivers. The method further comprises a sixth code segment for comparing measured values of signal quality or signal strength of transceivers in the present group of transceivers. The measured value of the signal strength may be a Received Signal Strength Indicator (RSSI). Furthermore, the computer program comprises a seventh code segment for establishing a link between the current node and the transceiver of the present group of transceivers for each transceiver of the present group of transceivers, if a criterion is met. A criterion that can be used is if the RSSI of the transceiver is higher than a threshold. Moreover, the computer program comprises an eighth code segment for from the present group of transceivers, selecting a transceiver with a best signal quality or a largest signal strength, such as a largest RSSI, as a current node. In addition, the computer program comprises a ninth code segment for repeating certain code segments until a preferred route of transmitting from the gateway to each transceiver has been established.

Further embodiments of the invention are defined in the dependent claims, wherein features for the second and subsequent aspects of the invention are as for the first aspect mutatis mutandis.

Some embodiments of the invention provide for that cancellation of an alarm is performed on a personal level instead of on a room level.

Some embodiments of the invention also provide for accurate measurement of position.

Some embodiments of the invention also provide for that dead-spots, i.e. places where no signal can be obtained, are avoided.

Some embodiments of the invention also provide for that the patients and/or the staff members can move around freely and still have access to their devices.

Some embodiments of the invention also provide for a dual use of the button for cancelling an alarm on the wireless staff member-specific device.

Some embodiments of the invention also provide for an extra safety feature for staff.

Some embodiments of the invention also provide for traceability of alarms and/or traceability of cancellations of alarms.

Some embodiments of the invention also provide for avoidance of unnecessary running around of staff, since the first staff member acknowledges the alarm and other staff members to not need to check the patient, who triggered the alarm.

Some embodiments of the invention also enable distinguishing between an emergency situation and a non-emergency situation.

Some embodiments of the invention also provide for redundancy in the system, and thus increased reliability of the system, since two gateways are used.

Some embodiments of the invention also provide for redundancy, and thus increased reliability of the system, since two different routes are selected and set-up.

Some embodiments of the invention also provide for that a route with the best signal quality is set-up. Thus, the system may be optimized.

Some embodiments of the invention also provide for that a redundant route with the second best signal quality is set-up.

Some embodiments of the invention also provide for self-healing of the system, e.g. when a transceiver is replaced with a new transceiver.

Some embodiments of the invention also provide for that no physical device configuration or user intervention is needed during replacement of a transceiver.

It should be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which embodiments of the invention are capable of will be apparent and elucidated from the following description of embodiments of the present invention, reference being made to the accompanying drawings, in which
Fig. 1 illustrates an area in which the wireless network system is set up and monitoring is performed;
Fig. 2 is an exemplary structure of a wireless networking system used for example in a patient monitoring, or in a system social alarm monitoring system or in a mentally disabled people monitoring system;
Fig. 3 is a detailed view of a patient-specific device;
Fig. 4 is a detailed view of a staff member-specific device;
Fig. 5 illustrates embodiments for communication between some units of the system;
Fig. 6 illustrates a transceiver according to some embodiments; and
Fig. 7 is a signalling diagram illustrating acknowledgments and cancellation signals sent between units of the system.

### DESCRIPTION OF THE PREFERRED EMBODIMENTS

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on an embodiment of the present invention applicable to a monitoring system and in particular to a method of setting up a monitoring system and/or to acknowledgement and cancelation of alarms in such a wireless network system. However, it will be appreciated that the invention is not limited to these application but may be applied to many other systems including for example monitoring of patients in their homes. Some examples of monitoring systems are: patient monitoring system, a social alarm monitoring system or mentally disabled people monitoring system, such as dementry, wandering people etc.

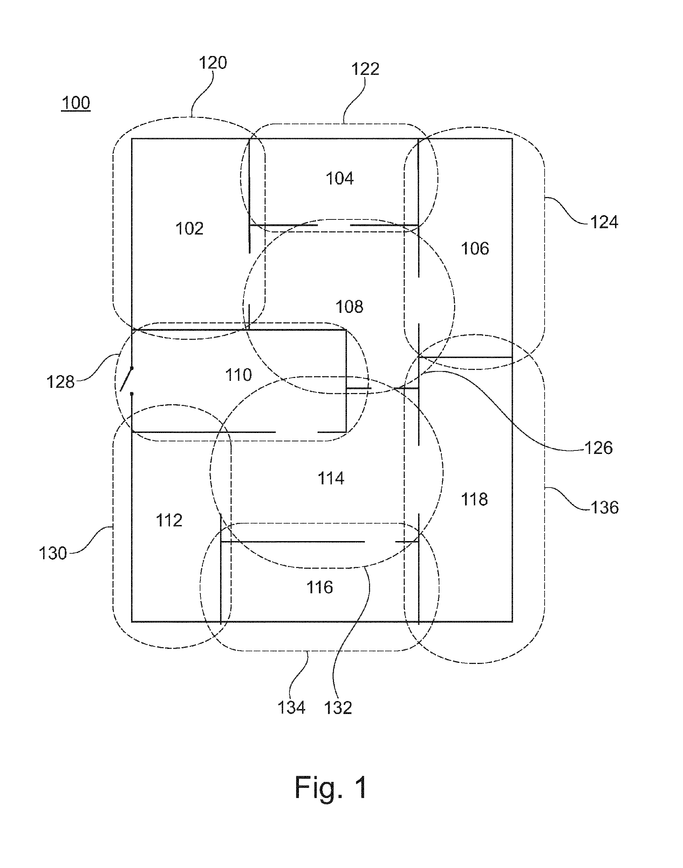

Fig. 1 shows an area in which monitoring is performed. Such an area may comprise different rooms 102-118. When a wireless monitoring system comprising wireless network transceivers is to be used, transceivers need to be placed so that all of the rooms to be monitored are covered. After placement of the transceivers, the different rooms should be mapped with the transceivers, so that each room is associated with at least one transceiver. A list of this mapping may then be stored and made available to a server. In some embodiments, transceivers are mapped to rooms 102-118 as illustrated in fig. 1. Each transceiver will in these embodiments cover a certain zone 120-136 of the monitored area.

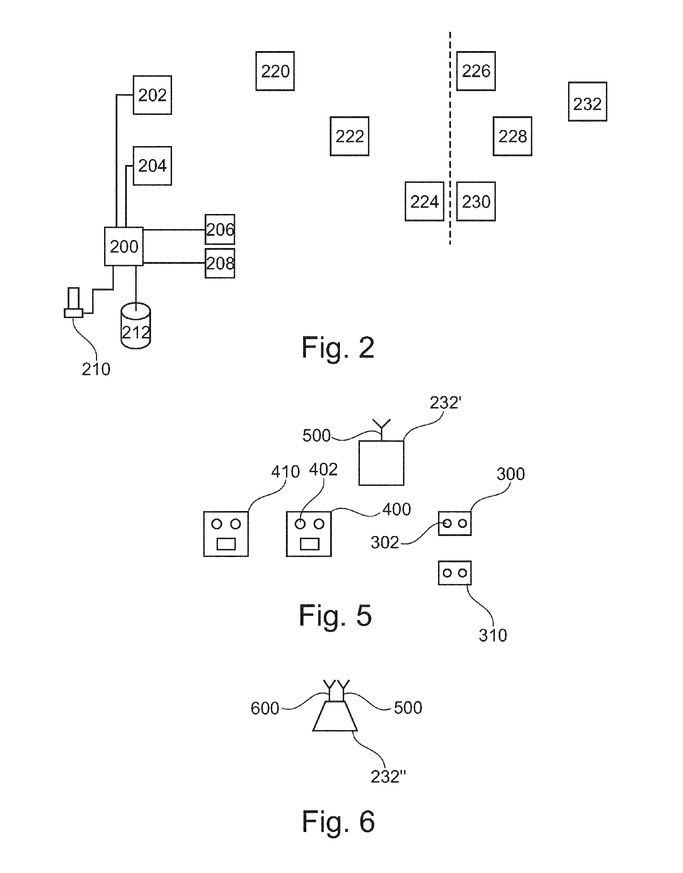

In an embodiment of the invention according to Fig. 2, a monitoring system comprises a server 200. In fig. 2, the server is connected to overhead screens 206, 208 and to an external storage unit 212. Furthermore, the server may also be connected to remote terminals 210 via a network, such as Internet. Moreover, the server is also connected to a first gateway 202 and a second gateway 204. The gateways 202, 204 may be in wireless connection with transceivers 220, 222, 224, 226, 228, 230, 232. The gateways 202, 204 are transceivers that communicate with the server 200.

In some embodiments, a preferred route of transmitting between the first gateway 202 and a transceiver is selected for each transceiver 220, 222, 224, 226, 228, 230, 232. The preferred route is directly between the first gateway 202 and the transceiver. Alternatively, the preferred route between the first gateway 202 and the transceiver is via at least one preferred route intermediate transceiver (as explained below). Whether the preferred route is direct or via another transceiver depends in one embodiment on a value of signal quality or signal strength, such as a Received Signal Strength Indicator (RSSI), of the transceiver measured at the first gateway or a value of signal quality or signal strength, such as an RSSI, of the first gateway measured at the transceiver. Thus, a route with the best signal quality is set up. In other embodiments, the selected preferred route is alternatively or in addition dependent on a Bit Error Rate (BER) and/ or a number of hops.

In some embodiments, also a secondary route of transmitting between the first gateway 202 and the transceiver is selected for each transceiver 220, 222, 224, 226, 228, 230, 232. The secondary route is either directly between the first gateway 202 and the transceiver or between the first gateway 202 and the transceiver via at least one secondary route intermediate transceiver. Whether the secondary route is direct or via another transceiver depends on a value of signal quality or signal strength, such as an RSSI, of the transceiver measured at the first gateway or a value of signal quality or signal strength, such as an RSSI, of the gateway measured at the transceiver. However, the at least one secondary route intermediate transceiver is different from the at least one preferred route intermediate transceiver. Thus, a redundant route with the second best signal quality is set up. Furthermore, by selecting two different routes, redundancy in the system is ensured.

In some embodiments, a preferred route of transmitting between the second gateway 204 and a transceiver is selected for each transceiver 220, 222, 224, 226, 228, 230, 232. The preferred route is either directly between the second gateway 204 and the transceiver or between the second gateway 204 and the transceiver via at least one preferred route intermediate transceiver. Whether the preferred route is direct or via another transceiver depends on a value of signal quality or a signal strength, such as an RSSI, of the transceiver measured at the second gateway 204 or a value of signal quality or signal strength, such as an RSSI, of the second gateway 204 measured at the transceiver. Also a secondary route of transmitting between the second gateway 204 and the transceiver is selected for each transceiver 220, 222, 224, 226, 228, 230, 232. The secondary route is either directly between the second gateway 204 and the transceiver or between the second gateway 204 and the transceiver via at least one secondary route intermediate transceiver, depending on a value of signal quality or signal strength, such as an RSSI, of the transceiver measured at the second gateway 204 or a value of signal quality or signal strength, such as an RSSI, of the second gateway 204 measured at the transceiver. The at least one secondary route intermediate transceiver is different from the at least one preferred route intermediate transceiver. Thus, there are two gateways for redundancy and both gateways have defined preferred and secondary routes. Thus, if anything happens to the first gateway 202, the second gateway 204 can directly replace the first gateway 202 and there will still be route redundancy in the system.

In some embodiments, the second gateway 204 is used for transmission instead of the first gateway 202, whenever the first gateway does not function correctly.

A method of setting up a wireless networking system is also disclosed. The wireless networking system may be used, for example in a monitoring system, such as patient monitoring, social alarm monitoring system or mentally disabled people monitoring system, such as dementry, wandering people etc.

The method may comprise mapping rooms, of a group of rooms, with transceivers of a first group of transceivers 220, 222, 224, 226, 228, 230, 232. As can be seen from fig.1, a transceiver covers a certain zone. As an example, one zone may cover a certain room. In the method, a gateway, e.g. the first gateway 202, is selected as a current node. From the current node, an installer command is broadcasted. The installer command may be "if this message is heard, then respond". After the installer command has been sent, the current node waits for a present group of transceivers to report as a response to the installer command. The transceivers of the present group should not already be linked to the gateway. The transceivers already linked to the gateway are excluded from the present group. The present group of transceivers belongs to the first group of transceivers, i.e. the present group of transceivers is a subgroup of the first group of transceivers. As an example, transceivers 220, 222 and 224 may have responded to the installer command. However, transceivers 226, 228, 230 and 232 may not have responded to the installer command. In the method, signal strength of each transceiver of the present group of transceivers is measured, e.g. signal strength of transceivers 220, 222 and 224 may be measured. Measured values of signal strength are compared. The measured and compared values may be Received Signal Strength Indicators (RSSI) of transceivers in the present group of transceivers. E.g. transceiver 220 may have the largest signal strength or RSSI, transceiver 222 may have fairly large signal strength or RSSI and transceiver 224 may have fairly low signal strength or RSSI. If a criterion is met for a transceiver, a link between the current node and the transceiver is established. Each transceiver of the present group of transceivers is checked against the criterion. A criterion to be used may be if the RSSI of the transceiver is higher than a certain or predetermined threshold. Another criterion that can be used is if the transceiver responded to the installer command or not. Thus, in the example, according to the first criteria (threshold), links between the gateway and e.g. transceivers 220 and 222 may be established and according to the second criteria (responded), links between the gateway and transceivers 220, 222 and 224 may be established. Furthermore, from the present group of transceivers, the transceiver with the largest RSSI is selected as a current node. E.g. transceiver 220 may be selected as a current node. Since this transceiver has an established link with the gateway, it can be selected as the current node, and an installer command may be forwarded to this transceiver from the gateway. The installer command may then be rebroadcasted from the current node. Since the current node now likely is closer to the transceivers that did not respond to the first installer command, new transceivers may respond to the rebroadcasted installer command. As an example, transceivers 226, 228 and 230 may respond to this rebroadcasted installer command. The transceivers 226, 228 and 230 will then use transceiver 220 as an intermediate transceiver and links will be established between transceiver 220 and transceivers 226, 228 and 230 in a similar manner as indicated above. This procedure will be repeated until a preferred route of transmitting from the gateway to each transceiver has been established.

In some embodiments, the method further comprises measuring a value of signal quality or signal strength, such as an RSSI, of the transceiver at the first gateway or measuring a value of signal quality or signal strength, such as an RSSI, of the gateway at the transceiver, for each transceiver. The method may also comprise, selecting a secondary route of transmitting between the first gateway and the transceiver either directly between the first gateway and the transceiver or between the first gateway and the transceiver via at least one secondary route intermediate transceiver depending on a value of the measured signal quality or signal strength, for each transceiver.

A computer-readable medium having embodied thereon a computer program for processing by a computer is also disclosed. The computer program comprises an optional first code segment for mapping a group of rooms with a first group of transceivers. The computer program also comprises a second code segment for selecting a gateway as a current node. The computer program further comprises a third code segment for from the current node broadcasting an installer command. Furthermore, the computer program comprises a fourth code segment for with the current node waiting for a present group of transceivers, transceivers of the present group not being linked to the gateway, the present group of transceivers belonging to the first group of transceivers, to report as a response to the installer command. Moreover, the computer program comprises a fifth code segment for receiving from the server 200 a measured signal strength of each transceiver of the present group of transceivers. The signal strength is measured by the transceiver 232 and a signal indicative of the signal strength is sent from the transceiver 232 to the server 200. In addition, the computer program comprises a sixth code segment for comparing measured values of signal strength, such as Received Signal Strength Indicators (RSSI), of transceivers in the present group of transceivers. Additionally, the computer program comprises a seventh code segment for, for each transceiver of the present group of transceivers, establishing a link between the current node and the transceiver of the present group of transceivers if a criterion, such as if the RSSI of the transceiver is higher than a threshold, is met. The method also comprises an eighth code segment for from the present group of transceivers, selecting a transceiver with the largest RSSI as a current node. The method may further comprise a ninth code segment for repeating until a preferred route of transmitting from the gateway to each transceiver has been established.

Optionally, the method may comprise a tenth code segment for measuring, for each transceiver, a value of signal quality or signal strength, such as an RSSI, of the transceiver at the first gateway or measuring, for each transceiver, a value of signal quality or signal strength, such as an RSSI, of the gateway at the transceiver and an eleventh code segment for selecting, for each transceiver, a secondary route of transmitting between the first gateway and the transceiver either directly between the first gateway and the transceiver or between the first gateway and the transceiver via at least one secondary route intermediate transceiver depending on the value of signal quality or signal strength.

In some embodiments, a transceiver of the plurality of transceivers can be replaced with a new transceiver by plug and play. Thus, the system can self-heal if a transceiver needs to be replaced, and thus no physical device configuration or user intervention is needed during replacement of a transceiver.

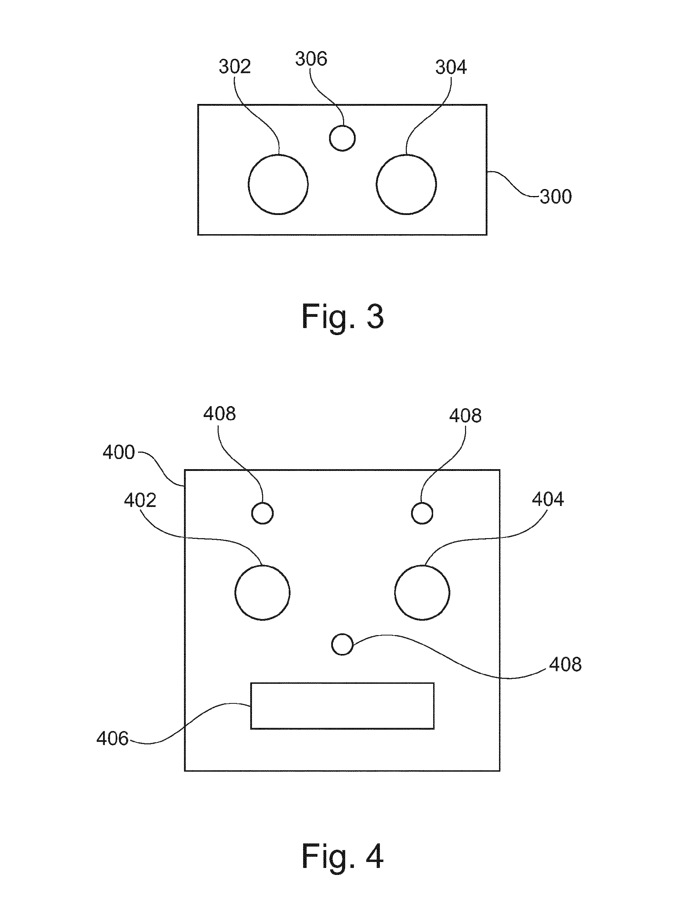

In some embodiments, the system also comprises one or several patient-specific devices 300. These patient-specific devices 300 are intended to be used by specific patients, i.e. each patient to be monitored is given a personal patient-specific device 300. A patient-specific device 300 is illustrated in fig. 3. The patient-specific device 300 may have a first button 302 and a second button 304. The first button 302 may be used for sending an alarm, i.e. the first button 302 may be used for emergencies. The second button 304 may be used for alerting the staff that some form of not urgent assistance is needed, i.e. the second button 304 may be used for situations that would not be classified as emergencies. The patient-specific device 300 may further have at least one LED 306 for indication of certain events, such as alarm sent and/or alarm acknowledged. Each patient-specific device 300 has a unique ID, which ID is linked to a specific patient. The linking data is preferably kept in a server database. The server database may be located in the external storage unit 212. Thus, there is no programming required at the patient-specific devices 300. Consequently, ease of use is achieved in mapping and altering the mapping of patient-specific devices 300 to patients.

In some embodiments, the system also comprises one or several staff member-specific devices 400. These staff member-specific devices 400 are intended to be used by specific staff members, such as physicians, nurses, care givers or janitors. Each staff member or at least a plurality of staff members is provided with a personal staff member-specific device 400. A staff member-specific device 400 is illustrated in fig. 4. The staff member-specific device 400 may have a first button 402 and a second button 404. In one embodiment, the staff-specific device 400 only has one button 402. The single button 402 facilitates that the device 400 can be operated without looking at it as there is only one button to press. Therefore the staff member-specific device 400 can be carried in a pocket of a staff member. This is advantageous, since staff members should not wear anything around their arms, while attending to patients and cancelling patient alarms. The first button 402 may be used for acknowledgement and/or cancellation of an alarm, i.e. the first button 402 may be used for acknowledging or cancelling emergencies. The second button 404 may be used as a personal alarm button, i.e. for alerting other staff members that the staff member carrying the staff member-specific device 400 is at danger and/or requires help. One of the first and second buttons 402, 404, e.g. the first button 402, may also be utilized for another function. Such another function may be updating of the staff member-specific device's position. This may be useful, e.g. if a staff member is in danger and running or moving away from the danger. The another function may instead or in addition be a personal alarm, i.e. an alarm for alerting other staff members that the staff member carrying the staff member-specific device 400 is at danger and/or requires help. One way of providing the first button 402 with a dual function is to provide the button with a first function if it is pressed shortly, i.e. pressed down during a period that is shorter than a certain time interval, e.g. 4 seconds, and then provide the button with a second function if the button is pressed down for a time period that is longer than a certain time interval, such as 4 seconds. The staff member-specific device 400 may further have at least one LED 408 for indication of certain events, such as acknowledgement received by a patient-specific device. Furthermore, the staff member-specific device may be equipped with a display for displaying relevant information, such as information related to a patient requiring assistance. Such patient-specific information may be advantageous to have, since it may facilitate assistance given to the patient, i.e. the staff member assisting the patient may be given information that will make it easier and/or faster for the staff member to decide what kind of assistance to give. In some embodiments, staff member-specific device 400 is able to give audible feedback e.g. when the patient device alarm is canceled. An audible feedback is advantageous, since the staff member will not be distracted, when attending to a patient and acknowledging the patient alarm. The staff member-specific device 400 may be provided with a loudspeaker or a buzzer for giving audible feedback to the staff member.

The system can keep a record of the alarm- or alert time and the event duration of all patient- and staff alarms. This record is kept at the central database and may be used to add to the staff planning schedules. The central database may be located in the external storage unit 212. It may be advantageous to identify exactly which member of staff attended to the patient and/or which member of staff created a high priority staff alarm. This is possible, since each staff member-specific device 400 has a unique ID, which ID may be linked to a specific staff member. In such case, mapping of staff member specific devices 400 to individual members of staff may be used. Such mapping occurs at the server 200 and does not require any programming of the staff member-specific devices 400. Consequently, ease of use is achieved in mapping and altering the mapping of staff member-specific devices 400 to staff members.

Fig. 5 shows some of the units of one embodiment of the invention. One of the units is a transceiver 232'. The transceiver 232' has in this embodiment an antenna 500 for sending and receiving signals from other units, such as patient-specific devices 300 and staff member-specific devices 400, of the system. In some embodiments, signals between a patient-specific device 300 and a staff member-specific device 400 may be conveyed via transceiver 232'. In other embodiments, signals between a patient-specific device 300 and a staff member-specific device 400 may be sent directly between the devices 300, 400. It is also possible that some signals are sent between a patient-specific device 300 and a staff member-specific device 400 directly and some signals between a patient-specific device 300 and a staff member-specific device 400 via transceiver 232'.

In some embodiments, illustrated in Fig. 6, the transceiver 232" has a first antenna 500 and a second antenna 600. The antennas 500, 600 are separated with a certain distance. Separation of antennas with a certain distance is also known as spatial diversity. In one embodiment, spatial diversity is achieved by switching between two or more antennas. In another embodiment, spatial diversity is achieved by using multiple transceivers, each transceiver having an antenna. This embodiment is advantageous, since retransmission of the signal when the weaker antenna has been in effect is avoided. Consequently, this embodiment is beneficial, because the reliability of the system is improved and the battery life of the portable devices is prolonged.

By the use of two antennas, the transceiver will be more reliable, since even if one of the antennas does not function properly, the transceiver 232" will still be able to send and receive signals. Furthermore, by choosing a separation distance of the antennas in a certain way, dead-spots, i.e. places where no signal can be obtained, can be avoided, since the physical distance between the antennas is related to the carrier frequency.

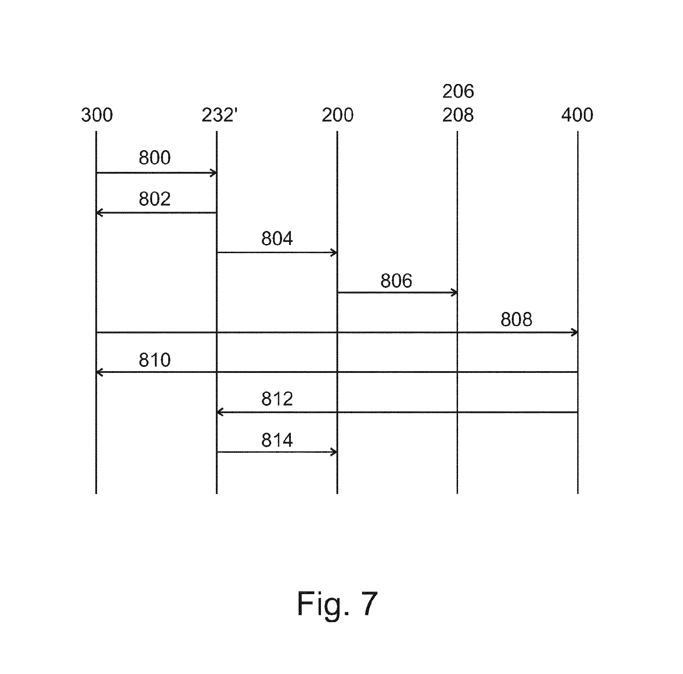

In one embodiment of the invention according to Fig. 7, an alarm signal 800 will be broadcasted to a network of fixed transceivers 232 from a patient-specific device 300 as a response to an event, where a patient has pressed an alarm button. The transceiver 232 having the best signaling path, typically the transceiver closest to the patient-specific device 300, will respond first.

In some embodiments, the patient-specific devices 300, 310 and/or the staff member-specific devices 400, 410 and/or the transceivers are wireless devices. In these embodiments, the listen-before-talk technique, which is part of the CSMA-CA messaging protocol between all wireless devices, prevents other transceivers 232 from responding to the same patient alarm message.

As a response to this signal, the transceiver 232' will send an acknowledgement signal 802 back to the patient-specific device 300.

At approximately the same time, the transceiver 232' will convey the alarm signal to the server 200 with a signal 804. The server 200 or a controller function thereof sends a control signal 806 to the plurality of overhead screens 206, 208. As a response to this control signal the patient alarm is annunciated audibly and/or visually at the overhead screens. A staff member will hear and/or see the alarm and attend to the patient, who sent the alarm. When the staff member, carrying the staff member-specific device 400, brings the staff member-specific device 400 close enough, e.g. closer than a threshold value, to the patient-specific device 300, the alarm can be cancelled by the staff member pressing a button, such as the first button 402 of the staff member-specific device 400. The threshold value may be a predetermined value. In some embodiments, the threshold value is adjusted by reduction of the transmit levels of the patient-specific devices 300 and/or the staff member-specific devices 400. In one embodiment, when the staff member is within close range, i.e. closer than the threshold value, of the patient, who sent the alarm, the staff member may momentarily press the button 402 of the staff member-specific device 400. This will open up a receiving time window, during which the staff member-specific device 400 can receive an alarm-cancellation request 808 from a patient-specific device 300. Within the staff member-specific device receiving time window, which may be between 1-30 seconds and preferably around 6 seconds, the staff member presses the button 302 of the patient-specific device 300, which has sent the alarm. The patient-specific device 300 will as a response to the button 302 being pressed, send a request 808 to cancel the patient alarm to the staff member-specific device 400. This is performed at reduced RF range. This request 808 is received by the staff member-specific device 400, if it is sent within the staff member-specific device receiving time window. The staff member-specific device 400 replies to the patient-specific device 300 with an acknowledgement signal 810 to cancel the patient alarm. As a response to this acknowledgement signal, the patient-specific device 300 exits an alarm state and can again send an alarm into the system. The staff member-specific device 400 now transmits a cancellation message 812 to a transceiver 232, which transceiver 232 will convey 814 the cancellation message to the server 200. The patient alarm is then cancelled at the server 200. Thereafter the annunciation of the alarm is cleared and when no more alarms are present, the audible indication is stopped. In this embodiment, the staff member-specific device 400 will not receive any alarms. The staff member-specific device 400 is used to cancel alarms from patient-specific devices 300 and/or other staff member-specific devices 400 by direct communication with the device in question. Furthermore, most of the communication is in this embodiment performed by the staff member-specific devices 400 and not by the patient-specific devices 300. This is advantageous, since the staff member-specific device 400 has much more battery capacity than the patient-specific device 300. Moreover, the staff member-specific device receiving time window can be made sufficiently short and the transmit levels of the patient-specific devices 300 and/or the staff member-specific devices 400 can be reduced sufficiently to assure that only the intended patient alarm, and thus not any alarms from patients nearby, is acknowledged.

In some embodiments, it is ensured that cancellation of an alarm can only be made when the staff member-specific device 400 is within a certain distance of the patient-specific device 300 by line of sight, e.g. by using an IR emitter and an IR receiver, which are operated as part of the patient alarm cancellation method. In these embodiments, the patient-specific device 300 has an IR emitter and the staff member-specific device 400 has an IR receiver. Alternatively the patient-specific device 300 has an IR receiver and the staff member-specific device 400 has an IR emitter.

In some embodiments, it is ensured that cancellation of an alarm can only be made when the staff member-specific device 400 is within a certain distance of the patient-specific device 300 by detection of a magnetic field emitted by a device, such as a magnet or a coil, with a corresponding device, such as a reed switch or a hall sensor. In these embodiments, the patient-specific device 300 has a magnet or a coil and the staff member-specific device 400 has a reed switch or a hall sensor. Alternatively the patient-specific device 300 has a reed switch or a hall sensor and the staff member-specific device 400 has a magnet or a coil.

In some embodiments, it is ensured that cancellation of an alarm can only be made when the staff member-specific device 400 is within a certain distance of the patient-specific device 300 by emission of an RF field, e.g. by the staff member-specific device 400, automatically or after pressing a button, which is detected at the patient-specific device 300 and/or powers a circuit at the patient-specific device 300.

In some embodiments, it is ensured that cancellation of an alarm can only be made when the staff member-specific device 400 is within a certain distance of the patient-specific device 300 by emission of an RF field, e.g. by the staff member-specific device 400, where the emitting device detects and/or communicates with a RFID tag, which is part of the patient-specific device 300. Alternatively, emission of an RF field by the patient-specific device 300 is detected by a RFID tag, which is part of the staff member-specific device 400.

In some embodiments, it is ensured that cancellation of an alarm can only be made when the staff member-specific device 400 is within a certain distance of the patient-specific device 300 by using NFC techniques.

In some embodiments, routing tables for the preferred route and the secondary route are established during setup of the system. The routing tables are thereafter downloaded into each transceiver 232. Thus, the transceivers 232 can perform the preferred routing and/or the redundant secondary routing retry autonomously.

In some embodiments, all communication to and/or from the staff member-specific device 400 and to and/or from patient-specific device 300 is initiated at the device itself. Such initiation may be performed automatically, e.g. by sending supervision messages for monitor presence, battery condition and trouble conditions. Alternatively or in addition, such initiation may be performed manually by pressing a button at the device.

In some embodiments, when the alarm-cancellation request 808 has been sent by the patient-specific device 300, and if no acknowledgement signal 810 is received from a nearby staff member-specific device 400, the patient-specific device 300 reverts back to the primary function of the button used, which is typically sending a low priority alert message into the system. This assures that derailing of the communication will not block sending alarms from other patient-specific devices 300 or from staff member-specific devices 400, and thus automatically brings the system back into synchronization. Such situations may occur e.g. when staff member-specific devices 400 are momentarily out of range while alarm/alert messages are being sent.

As used herein, the singular forms "a", "an" and "the" are intended to include the plural forms as well, unless expressly stated otherwise. It will be further understood that the terms "includes," "comprises," "including" and/or "comprising," when used in this specification, specify the presence of stated features, integers, steps, operations, elements, and/or components, but do not preclude the presence or addition of one or more other features, integers, steps, operations, elements, components, and/or groups thereof. It will be understood that when an element is referred to as being "connected" or "coupled" to another element, it can be directly connected or coupled to the other element or intervening elements may be present. Furthermore, "connected" or "coupled" as used herein may include wirelessly connected or coupled. As used herein, the term "and/or" includes any and all combinations of one or more of the associated listed items.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

As will be appreciated by one of skill in the art, the present invention may be embodied as a device, a system, a method or a computer program product. Accordingly, the present invention may take the form of an entirely hardware embodiment, a software embodiment or an embodiment combining software and hardware aspects all generally referred to herein as a "circuit" or "module." Furthermore, the present invention may take the form of a computer program product on a computer-usable storage medium having computer-usable program code embodied in the medium. Any suitable computer readable medium may be utilized including hard disks, CD-ROMs, optical storage devices, a transmission media such as those supporting the Internet or an intranet, or magnetic storage devices.

Embodiments of the present invention are described herein with reference to flowchart and/or block diagrams. It will be understood that some or all of the illustrated blocks may be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer-readable memory that can direct a computer or other programmable data processing apparatus to function in a particular manner, such that the instructions stored in the computer-readable memory produce an article of manufacture including instruction means which implement the function/act specified in the flowchart and/or block diagram block or blocks. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions/acts specified in the flowchart and/or block diagram block or blocks.

Although a loop in a method may be described as an infinite loop, it should be understood that such a loop can be ended also after a certain number of loops.

Although some code segments may have been named and/or described as different code segments, some of the code segments, i.e. first to 11^{th} code segment, may actually be the same code segment.

It is to be understood that the functions/acts noted in the diagrams may occur out of the order noted in the operational illustrations. For example, two blocks shown in succession may in fact be executed substantially concurrently or the blocks may sometimes be executed in the reverse order, depending upon the functionality/acts involved. Although some of the diagrams include arrows on communication paths to show a primary direction of communication, it is to be understood that communication may occur in the opposite direction to the depicted arrows.

The present invention has been described above with reference to specific embodiments. However, other embodiments than the above described are equally possible within the scope of the invention. Different method steps than those described above, performing the method by hardware or software, may be provided within the scope of the invention. The different features and steps of the invention may be combined in other combinations than those described. The scope of the invention is only limited by the appended patent claims.

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto, the invention may be practiced otherwise than as specifically described and claimed. The present invention is directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present invention.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases and disjunctively present in other cases.

Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc. As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements.

This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B." or, equivalently "at least one of A and/or B") can refer, in one embodiment, to at least one, optionally including more than one, A, with no B present (and optionally including elements other than B); in another embodiment, to at least one, optionally including more than one, B. with no A present (and optionally including elements other than A), in yet another embodiment, to at least one, optionally including more than one, A, and at least one, optionally including more than one, B (and optionally including other elements), etc. It should also be understood that, unless clearly indicated to the contrary, in any methods claimed herein that include more than one step or act, the order of the steps or acts of the method is not necessarily limited to the order in which

## Claims

1. A wireless network system comprising:
a first gateway (202);
optionally a second gateway (204);
a plurality of transceivers (220, 222, 224, 226, 228, 230, 232); and
wherein a preferred route of transmitting between said first gateway (202) and a transceiver is selected for each transceiver (220, 222, 224, 226, 228, 230, 232),
said preferred route being either directly between said first gateway (202) and said transceiver or
between said first gateway (202) and said transceiver via at least one preferred route intermediate transceiver depending on a value of signal quality or
signal strength, such as a Received Signal Strength Indicator (RSSI), of said transceiver measured at said first gateway (202) or a value of signal quality or
signal strength, such as an RSSI, of said first gateway (202) measured at said transceiver.

2. The system of claim 1, wherein a secondary route of transmitting between said first gateway (202) and said transceiver is selected for each transceiver (220, 222, 224, 226, 228, 230, 232), said secondary route being either directly between said first gateway (202) and said transceiver or between said first gateway (202) and said transceiver via at least one secondary route intermediate transceiver depending on a value of signal quality or signal strength, such as an RSSI, of said transceiver measured at said first gateway (202) or a value of signal quality or signal strength, such as an RSSI, of said first gateway (202) measured at said transceiver and
wherein said at least one secondary route intermediate transceiver is different from said at least one preferred route intermediate transceiver.

3. The system of claim 2, further comprising said second gateway (204), and
wherein a preferred route of transmitting between said second gateway (204) and a transceiver is selected for each transceiver (220, 222, 224, 226, 228, 230, 232), said preferred route being either directly between said second gateway (204) and said transceiver or between said second gateway (204) and said transceiver via at least one preferred route intermediate transceiver, depending on a value of signal quality or signal strength, such as an RSSI, of said transceiver measured at said second gateway (204) or a value of signal quality or signal strength, such as an RSSI, of said second gateway (204) measured at said transceiver; and
wherein a secondary route of transmitting between said second gateway (204) and said transceiver is selected for each transceiver (220, 222, 224, 226, 228, 230, 232), said secondary route being either directly between said second gateway (204) and said transceiver or between said second gateway (204) and said transceiver via at least one secondary route intermediate transceiver, depending on a value of signal quality or signal strength, such as an RSSI, of said transceiver measured at said second gateway (204) or a value of signal quality or signal strength, such as an RSSI, of said second gateway (204) measured at said transceiver, said at least one secondary route intermediate transceiver being different from said at least one preferred route intermediate transceiver.

4. The system of claim 3, wherein said second gateway (204) is used for transmission instead of said first gateway (202), whenever said first gateway (202) does not function correctly.

5. The system of any of the proceeding claims, wherein at least one transceiver has two antennas (500, 600).

6. The system of any of claims 2-5, wherein said preferred route and/or said secondary route are utilized for transmission of alarms and/or acknowledgement messages and/or cancellation of said alarms.

7. The system of any of the proceeding claims, further comprising a wireless patient-specific device (300) and/or a wireless staff-specific device (400) and/or wherein said transceivers (220, 222, 224, 226, 228, 230, 232) are wireless network transceivers.

8. The system of any of claims 2-7, further comprising:
a server (200);
an overhead screen (206); and
wherein said first and second gateways (202, 204) are connected to said server (200) and wherein said server (200) is connected to said overhead screen (206).

9. The system of any of the proceeding claims, wherein a transceiver of said plurality of transceivers (220, 222, 224, 226, 228, 230, 232) can be replaced with a new transceiver by plug and play.

10. A method of setting up a wireless network system comprising:
a) optionally mapping rooms, of a group of rooms, with transceivers of a first group of transceivers;
b) selecting a gateway as a current node;
c) from said current node broadcasting an installer command;
d) with said current node waiting for a present group of transceivers, transceivers of said present group not being linked to said gateway, said present group of transceivers belonging to said first group of transceivers, to report as a response to said installer command;
e) measuring a value of signal quality or signal strength of each transceiver of said present group of transceivers;
f) comparing measured values of signal quality or signal strength, such as Received Signal Strength Indicators (RSSI), of transceivers in said present group of transceivers;
g) for each transceiver of said present group of transceivers, establishing a link between said current node and said transceiver of said present group of transceivers if a criterion, such as if said RSSI of said transceiver is higher than a threshold, is met;
h) from said present group of transceivers, selecting a transceiver with a best signal quality or a largest signal strength, such as a largest RSSI, as a current node;
i) repeating steps c) to h) until a preferred route of transmitting from said gateway to each transceiver has been established.

11. The method of claim 10, further comprising:
j) for each transceiver, measuring a value of signal quality or signal strength, such as an RSSI, of said transceiver at said gateway or measuring a value of signal quality or signal strength, such as an RSSI, of said gateway at said transceiver;
k) for each transceiver , selecting a secondary route of transmitting between said gateway and said transceiver either directly between said gateway and said transceiver or between said gateway and said transceiver via at least one secondary route intermediate transceiver depending on a value of said measured signal quality or signal strength;

12. A computer-readable medium having embodied thereon a computer program for processing by a computer, the computer program comprising:
an optional first code segment for mapping a group of rooms with a first group of transceivers;
a second code segment for selecting a gateway as a current node;
a third code segment for broadcasting an installer command from said current node;
a fourth code segment for, with said current node,
waiting for a present group of transceivers,
transceivers of said present group not being linked to said gateway, said present group of transceivers belonging to said first group of transceivers, to report as a response to said installer command;
a fifth code segment for receiving from the server 200 a measured value of signal quality or signal strength of each transceiver of said present group of transceivers;
a sixth code segment for comparing measured values of signal quality or signal strength, such as Received Signal Strength Indicators (RSSI), of transceivers in said present group of transceivers;
a seventh code segment for, for each transceiver of said present group of transceivers, establishing a link between said current node and said transceiver of said present group of transceivers, if a criterion, such as if said RSSI of said transceiver is higher than a threshold, is met;
an eighth code segment for, from said present group of transceivers, selecting a transceiver with a best signal quality or a largest signal strength,
such as a largest RSSI, as a current node;
a ninth code segment for repeating code segments until a preferred route of transmitting from said gateway to each transceiver has been established.

13. The computer-readable medium having embodied thereon a computer program for processing by a computer of claim 12, the computer program further comprising:
a tenth code segment for measuring, for each transceiver, a value of signal quality or signal strength, such as an RSSI, of said transceiver at said gateway or measuring, for each transceiver, a value of signal quality or signal strength, such as an RSSI, of said gateway at said transceiver;
an eleventh code segment for selecting, for each transceiver, a secondary route of transmitting between said gateway and said transceiver either directly between said gateway and said transceiver or between said gateway and said transceiver via at least one secondary route intermediate transceiver depending on said value of signal quality or signal strength.

14. Use of a system according to any of claims 1 to 9; and/or a method according to any of claims 10 to 11; and/or a computer-readable medium according to any of claims 12 to 13, in a monitoring system, such as, a patient monitoring system, or a social alarm monitoring system or a mentally disabled people monitoring system, such as dementry, wandering people etc.
